# EUROPÄISCHE PATENTSCHRIFT

(11) **EP 3 381 250 B1**
(45) Veröffentlichungstag und Bekanntmachung des Hinweises auf die Patenterteilung: **03.06.2020**
(21) Anmeldenummer: 17020129.7
(22) Anmeldetag: 31.03.2017
(51) Int. Cl.: A01B 1/02, A01B 79/00

(54) **GARTENGERÄT ZUR BODENBEARBEITUNG UND SAAT-BZW. PFLANZVERFAHREN MIT HILFE EINES SOLCHEN GARTENGERÄTS**
GARDENING DEVICE FOR CULTIVATING SOIL, AND SOWING OR PLANTING METHOD WITH THE AID OF SUCH GARDENING DEVICE
OUTIL DE JARDINAGE DESTINÉ AU TRAITEMENT DU SOL ET PROCÉDÉ DE PLANTATION OU DE SEMIS À L'AIDE D'UN TEL OUTIL DE JARDINAGE

(43) Veröffentlichungstag der Anmeldung: 03.10.2018
(73) Patentinhaber: Scheppach Fabrikation von Holzbearbeitungsmaschinen GmbH, 89335 Ichenhausen (DE)
(72) Erfinder: Bindhammer, Markus, 86316 Friedberg (DE)
(74) Vertreter: Patentanwälte Munk

(56) Entgegenhaltungen:
- CA-A1- 2 836 642
- CN-A- 104 737 638
- CN-A- 105 594 321
- DE-U1-202008 007 479
- US-A- 5 975 601
- US-A1- 2008 301 879
- US-A1- 2010 321 021

## Beschreibung

Die Erfindung betrifft ein Gartengerät zur Bodenbearbeitung gemäß dem Oberbegriff des Anspruchs 1, sowie ein mit Hilfe eines solchen Gartengeräts durchgeführtes Saat- bzw. Pflanzverfahren.

Aus der Schrift DE 10 2009 010 579 A1 ist ein System bekannt, das eine Pflanze beim Wachsen überwacht. Um den Boden dagegen vorab daraufhin zu kontrollieren, ob er für eine bestimmte Pflanze geeignet ist, sind bereits Bodensonden bekannt, wie beispielsweise die in der deutschen Patentanmeldung DE 10 2012 106 841 A2 offenbarte Bodenfeuchtigkeitsmesssonde. Die dort erläuterte Messsonde weist einen kapazitiv arbeitenden Sensor auf, der sich in einem Gehäuse mit einer Fensterausnehmung befindet. In der Fensterausnehmung sitzt ein Zugangselement, welches mit einem hydrophilen Material imprägniert ist. Je nachdem, wie viel Wasser das umgebende Erdreich hat, zieht das Zugangselement viel oder wenig Wasser an und dient als Elektrizitätsmedium für die kapazitive Messeinrichtung. Die Bodenfeuchtigkeitsmesssonde ist jedoch zumindest für den Hobbygärtner nicht nur zu teuer, sondern auch in der Handhabung zu aufwändig.

Es gibt ferner bereits Gartensensoren, die in den Erdboden eingebracht werden und dort die Feuchte, Lichtintensität und die Temperatur messen und beispielsweise auf Handy eines Benutzers übertragen, so dass dieser über die momentan herrschenden Bedingungen für Aussaat oder Pflanzungen, aber auch während der Wachstumsphase seiner Pflanzen informiert ist. Während mit derartigen Sensoren der gärtnerische Erfolg gesteigert werden kann, bleibt die Handhabung jedoch mühsam.

Der Trend geht daher dahin, die Sensoren bereits in die vom Gärtner zu verwendenden Geräte zu integrieren. Ein solches Gerät offenbart die US-Patentanmeldung US 5,975,601 A, nämlich eine Gartenschaufel, die einstückig aus einem geschmolzenen Kompositmaterial, wie beispielsweise glasverstärktem Nylon, gefertigt ist.

So offenbart die internationale Patentanmeldung WO 2016 118 000 A1 ein Düngereinbringungsgerät, mit dem Löcher in den Boden gestochen und dann darin der Dünger eingebracht werden kann. Zudem weist das Gerät einen Temperatur- und einen Ph-Sensor auf, um die Temperatur oder den Ph-Wert des Bodens zu ermitteln, bevor der Dünger in den Boden eingebracht wird. Ein derartiges Düngereinbringungsgerät ist jedoch für den gärtnerischen Einsatz außerhalb der industriellen Landwirtschaft sehr teuer und vom eigentlichen Anwendungszweck her zu speziell, als dass die verwendeten Messfühler ihren vollen Nutzen ausspielen könnten.

Die kanadische Patentanmeldung CA 2 836 642 A1 zeigt dagegen eine Gartenschaufel mit einem Feuchte- und einem Nitratgehaltssensor am Schaufelblatt. Am Schaufelstiel ist dabei ein Display vorgesehen. Ferner weist die Schaufel einen Microchip auf, der die Messergebnisse an entfernte Geräte wie Computer oder Mobiltelefone weitersenden kann. Der Nitratsensor ist dabei in eine Silikatmembran eingebettet und befindet sich hinterhalb des Schaufelblatts, wobei Diphenylamin mit Nitratpartikeln in der Silikatmembran reagiert und sich dadurch blau färbt und der Sensor eine spektrophotometrische Erfassung der Färbung und damit der Nitratmenge durchführt. Der Feuchtigkeitssensor befindet sich dagegen auf der Vorderseite des Schaufelblatts und besteht aus mikroskopischen Platten mit einer Vielzahl wasserdruckempfindlichen Scheiben, die bei Kontakt mit Wasser anschwellen und bei Erreichen einer kritischen Länge einen Aktuator kontaktieren, der ein der aufgenommenen Wassermenge entsprechendes Signal abgibt.

Hiervon ausgehend ist es Aufgabe der vorliegenden Erfindung, den Aufbau des Gartengeräts so zu verbessern, dass sich bei geringen Kosten eine hohe Robustheit und ein damit auf einfache Weise und mit hoher Zuverlässigkeit durchführbares Saat- bzw. Pflanzverfahren ergibt.

Diese Aufgabe wird hinsichtlich des Gartengeräts mit den Merkmalen eines Anspruchs 1 gelöst, hinsichtlich des Saat- bzw. Pflanzverfahrens mit den Merkmalen des Anspruchs 15.

Das erfindungsgemäße Gartengerät zur Bodenbearbeitung weist einen Handgriffabschnitt und einen daran angebrachten Bodenkontaktabschnitt auf. Vorzugsweise ist das Gartengerät als Gartenschaufel ausgebildet, so dass der Bodenkontaktabschnitt die Form eines Schaufelblatts hat. Weiterhin weist das erfindungsgemäße Gartengerät eine Stromversorgung, eine Anzahl Erfassungseinrichtungen zur Erfassung einer Anzahl von Bodeneigenschaften entsprechender Größen, eine Recheneinheit zur Berechnung der Bodeneigenschaften aus der den Bodeneigenschaften entsprechenden Anzahl von Größen, und einer Ausgabeeinrichtung zur Ausgabe der Bodeneingenschaften und/oder auf den Bodeneigenschaften basierenden Angaben auf. Der Bodenkontaktabschnitt trägt Elektroden, über welche die Anzahl der den Bodeneigenschaften entsprechenden Größen erfasst werden. Ferner weist das Gartengerät eine Nährstofferfassungseinrichtung zur Erfassung einer Anzahl einem Nährstoffgehalt im Boden entsprechender Größen auf.

Das erfindungsgemäße Gartengerät zeichnet sich dabei dadurch aus, dass der Bodenkontaktabschnitt aus Kunststoff besteht, vorzugsweise aus faserverstärktem Kunststoff gefertigt ist. Weiterhin ist die Nährstofferfassungseinrichtung dabei so aufgebaut, dass sie eine elektrische Leitfähigkeit des Bodens als Maß für den Nährstoffgehalt erfasst. Dazu umfasst sie zumindest zwei der Elektroden, die voneinander beabstandet auf dem Bodenkontaktabschnitt des Gartengeräts angebracht sind. Über diese beiden Leitfähigkeitsmesselektroden kann die elektrische Leitfähigkeit des Bodens gemessen werden, welche umso größer ist, je mehr Nährstoffionen sich im Boden befinden und daher ein Maß für den Nährstoffgehalt des Bodens bildet. Die beiden Leitfähigkeitsmesselektroden bestehen dabei aus Chemisch Nickel oder umfassen eine Schicht aus Chemisch Nickel.

Dadurch, dass das Schaufelblatt aus einem nicht leitenden Material besteht, lassen sich die Elektroden einfach auf dem Bodenkontaktabschnitt auftragen, ohne dass aufwändige Maßnahmen zur Isolierung der einzelnen Elektroden voneinander getroffen werden müssten. Manche Kunststoffe wie ABS weisen zudem eine relativ hohe Festigkeit und Abriebarmut auf, die sich auch zum Einsatz als Gartengerät gut eignet. In besonderem Maße gilt dies jedoch für faserverstärkte Kunststoffe. Bevorzugt ist daher, wenn der Bodenkontaktabschnitt vollständig aus faserverstärktem Kunststoff gefertigt ist, wobei sich insbesondere glasfaserverstärkter Kunststoff gut eignet, da dieser neben einer hohen Festigkeit und Abriebarmut auch noch stark dielektrisch wirkt. Es wäre auch denkbar, nur Teile des Schaufelblatts bzw. des Bodenkontaktabschnitts im Bereich der Elektroden aus Kunststoff auszubilden. Insbesondere eine aus Chemisch Nickel bestehende Schicht lässt sich bei einem aus Kunststoff wie z.B. ABS, GFK oder CFK bestehenden Bodenkontaktabschnitt sehr gut abscheiden.

Vorzugsweise sind die Elektroden auf der Oberfläche des Bodenkontaktabschnitts, also z.B. des Schaufelblatts aufgebracht, so dass sie mit dem Boden bzw. der Erde in Kontakt bringbar sind.

Eine weitere der Anzahl Erfassungseinrichtungen kann z.B. eine Feuchtigkeitserfassungseinrichtung zur Erfassung einer Anzahl einer Bodenfeuchtigkeit entsprechender Größen sein, wobei dann die Recheneinheit zur Berechnung der Bodenfeuchtigkeit aus der der Bodenfeuchtigkeit entsprechenden Anzahl von Größen eingerichtet ist und die Ausgabeeinrichtung zur Ausgabe der Bodenfeuchtigkeit und/oder auf der Bodenfeuchtigkeit basierenden Angaben wie beispielsweise ob sich die Bodenfeuchtigkeit für eine bestimmte Pflanze eignet.

Die hohe Dielektrizität des glasfaserverstärkten Kunststoffs wirkt sich insbesondere dann positiv aus, wenn z.B. die vorstehend angesprochene Feuchtigkeitserfassungseinrichtung zwei der Elektroden umfasst, welche nach Art eines Plattenkondensators voneinander so beabstandet auf dem Bodenkontaktabschnitt angebracht sind, dass eine Kapazität des Plattenkondensators mit Erde als Dielektrikum beeinflusst wird, wenn die Erde mit dem Bodenkontaktabschnitt im Bereich zwischen diesen Kondensatorelektroden kontaktiert wird. Dadurch gelingt ein kostengünstiger, aber zuverlässig arbeitender Aufbau der Feuchtigkeitserfassungseinrichtung.

Die Kondensatorelektroden der Feuchtigkeitserfassungseinrichtung können vorteilhaft aus einem leitfähigen Material wie Kupfer oder einer Kupferlegierung bestehen und gegenüber der Umgebung luft- und feuchtigkeitsdicht versiegelt auf dem Bodenkontaktabschnitt aufgebracht sein. Darum sind die Elektroden nicht nur kostengünstig herstellbar, sondern auch gegen Korrosion oder Oxidation geschützt, so dass das Elektrodenmaterial nicht aufwendig oberflächenbehandelt oder aus teuren Elementen oder Legierungen bestehen muss. Durch die Versiegelung wird zudem verhindert, dass die Elektroden in direktem Kontakt mit dem Erdboden treten, so dass ein unerwünschter Stromfluss vermieden wird. Möglich wäre es ferner, die beiden Kondensatorelektroden versetzt zueinander auf Vorder- und Rückseite des Schaufelblatts bzw. des Bodenkontaktabschnitts aufzubringen. Es wäre jedoch auch denkbar, die Kondensatorelektroden in das Innere des Kunststoffs einzubetten oder sogar in das Gewebe der Faserverstärkung einzuflechten.

Die beiden Kondensatorelektroden befinden sich dabei vorteilhaft örtlich zwischen den beiden Leitfähigkeitsmesselektroden der Nährstofferfassungseinrichtung, so dass sie so nahe beieinander angeordnet sein können, dass sie einen Plattenkondensator ausbilden können, wohingegen die Entfernung der beiden Leitfähigkeitsmesselektroden der Nährstofferfassungseinrichtung, die ja einen Stromfluss zwischen sich erlauben sollen, weiter voneinander beabstandet sein können.

Vorteilhaft ist jede Nickelschicht der Leitfähigkeitsmesselektroden dabei mit einer Goldschicht bedeckt, welche nicht nur die Oxidation des Nickels verhindert, sondern auch einen gut leitfähigen Kontakt zum Erdreich herstellen kann. Die beiden Leitfähigkeitsmesselektroden bestehen also besonders bevorzugt aus Chemisch Nickel/Sudgold. Die Nickelschicht kann dabei beispielsweise zwischen 4 und 7 µm dick sein, die darauf angebrachte Goldschicht zwischen 0,05 und 0,1 µm.

Mittels der Goldschicht soll auch verhindert werden, dass sich die Leitfähigkeitsmesselektroden, also der Nickel, zersetzt und dabei für die Pflanzen giftige Ionen freigesetzt werden.

Das erfindungsgemäße Saat- bzw. Pflanzverfahren kann dann die folgenden Schritte umfassen:
Ermittlung eines für eine bestimmte, auszusäende oder zu pflanzende Gartenpflanze oder Gemüseart günstigen Sollwerts für die Bodenfeuchtigkeit und den Nährstoffgehalt im Boden, an einer zur Aussaat bzw. zum Pflanzen angedachten Stelle das Ermitteln eines Istwerts der Bodenfeuchtigkeit und des Nährstoffgehalts im Boden, anschließend Vergleich der Sollwerte mit den Istwerten und, falls der Vergleich positiv ausfällt, Aussaat bzw. Pflanzen der Gartenpflanze oder Gemüseart, wobei zumindest das Ermitteln des Istwerts für die Bodenfeuchtigkeit mit Hilfe des erfindungsgemäßen Gartengeräts durchgeführt wird, welches dazu an der zur Aussaat bzw. zum Pflanzen vorgesehenen Stelle in den Boden gesteckt wird und welches dann im positiven Vergleichsfall gleich zum Erstellen des Lochs für den Samen oder die Pflanze bei der Aussaat bzw. beim Pflanzen dienen kann.

Vorteilhaft ist die Recheneinheit dabei dazu eingerichtet, aus der Kapazität des Plattenkondensators und/oder aus einer aus der Kapazität des Plattenkondensators abgeleiteten Größe als Eingangsgröße eine die Bodenfeuchtigkeit wiedergebende Ausgangsgröße zu errechnen. Dazu kann die Recheneinheit eine in ihrem Speicher abgelegte Lookup-Tabelle oder dergleichen enthalten, aus der der Zusammenhang hervorgeht.

Je nach Stärke des Dielektrikums zwischen den Kondensatorelektroden der Feuchtigkeitserfassungseinrichtung, also je nach Bodenfeuchtigkeit ergibt sich dabei ein anderer Wert für die Kapazität des durch diese beiden Kondensatorelektroden gebildeten Plattenkondensators. Die Kapazität kann dabei erfasst werden. Deutlich einfacher und genauer lässt sich jedoch eine Schwingungsfrequenz als Maß für die Kapazität des Plattenkondensators bzw. die Dielektrizität der Erde und damit die Bodenfeuchte ermitteln. Vorteilhaft weist die Feuchtigkeitserfassungseinrichtung daher einen mit dem Plattenkondensator zu einer Oszillatorschaltung zusammengeschalteten Schwingungserzeuger auf. Einfach auswerten lässt sich die Oszillatorschaltung, wenn diese als Kippschwinger binäre Ausgangssignale abgibt. Dazu kann der Schwingungserzeuger als Schmitt-Trigger ausgebildet sein. Die Frequenz der durch Kontakt mit der Erde erzeugten Schwingung kann dann anstatt oder zusätzlich zur Kapazität des Plattenkondensators der Recheneinheit als Eingangsgröße für die Bodenfeuchtigkeit zugeführt werden.

Vorteilhaft ist der Bodenkontaktabschnitt dabei ferner auswechselbar und insbesondere ohne Werkzeug auswechselbar an dem Handgriffabschnitt angebracht, beispielsweise über einen Bajonettverschluss oder die aus dem Gartenbereich hinlänglich bekannten Steckverbindungen, z.B. von der Fa. Gardena. Für die elektrische Kontaktierung der Elektroden könnte dabei eine zusätzliche Steckverbindung oder dergleichen vorgesehen sein. Das Schaufelblatt oder der als ein anderes Arbeitsgerät gestalteter Bodenkontaktabschnitt kann dann einfach ausgetauscht werden, wenn es abgenutzt ist, ohne dass der die Elektronik enthaltende Handgriffabschnitt erneuert werden muss.

Entsprechend umfasst der Handgriffabschnitt vorteilhaft einen Stiel, in dem ein Microcontroller der Recheneinheit und eine Anzahl Batterien der Stromversorgung untergebracht sind, z. B. ein 9-V- Block oder ein aufladbarer Akku, wobei an dem Stiel ein Ein/Aus-Schalter und vorzugsweise auch ein Display der Ausgabeeinrichtung angeordnet ist.

Vorzugsweise weist der Handgriffabschnitt dabei an seinem dem Bodenkontaktabschnitt abgewandten Ende eine abnehmbare Verschlusskappe auf, über die ein Batterieaufnahmefach im Inneren des Stiels zugänglich ist, so dass die Batterie entnommen bzw. getauscht werden kann, wenn sie entladen ist. Die Verbindung der Verschlusskappe mit dem Handgriffabschnitt ist dabei vorteilhaft wasserdicht ausgeführt, so dass kein Schmutz oder Wasser in das Innere des Batteriefachs gelangen kann. Das gilt natürlich für die weitere, im Inneren des Stiels bevorzugt in einer separaten Kammer aufgenommene Elektronik, wie beispielsweise den Microcontroller der Recheneinheit, wobei diese Kammer auch fest verschlossen sein kann.

Gemäß einer weiteren bevorzugten Ausbildung könnte ein aufladbarer Akkumulator als Batterie vorgesehen sein, wobei das Gartengerät an seinem Handgriffabschnitt und dort bevorzugt an seinem den Bodenkontaktabschnitt abgewandten Ende eine entsprechende Anschlussbuchse für ein Ladegerät aufweist. Weiterhin denkbar wäre auch ein kontaktloses Akku-Ladesystem, insbesondere ein induktives Ladesystem nach Art einer elektrischen Zahnbürste, so dass dann die Batterie fest verbaut und im Inneren des Stiels eingesiegelt sein könnte.

In besonders bevorzugter Weiterbildung der Ausführung des Gartengeräts mit einem auswechselbar an dem Handgriffabschnitt angebrachten Bodenkontaktabschnitt weist das Gartengerät eine Mehrzahl unterschiedlich geformter, jeweils mit den beiden Kondensatorelektroden versehener und auf den Handgriffabschnitt passender Bodenkontaktabschnitte auf. Das Gartengerät kann dann zu vielseitigen Einsatzzwecken verwendet werden. Beispielsweise könnte einer der Bodenkontaktabschnitte als ein Schaufelblatt, ein weiterer Bodenkontaktabschnitt als eine Kelle und wieder ein anderer Bodenkontaktabschnitt als eine Hacke ausgebildet sein etc..

Weiterhin vorteilhaft kann die Ausgabevorrichtung ein Kommunikationsmodul umfassen, mit dem drahtlos Daten auf ein externes Gerät, wie z.B. ein Smartphone oder einen PC übertragen werden können. Das Kommunikationsmodul kann dabei beispielsweise als Bluetooth-Funkmodul oder als WLAN-Funkmodul ausgebildet sein und alternativ oder ergänzend zu dem Display am Stiel des Handgriffsabschnitts vorgesehen sein. Damit gelingt nicht nur ein besserer Überblick über die ermittelten Messwerte und die für die Aussaat oder Pflanzung daraus folgenden Konsequenzen, also ob die Stelle, an der das Gartengerät in den Boden gesteckt worden ist, sich für die Aussaat bzw. Pflanzung eignet oder nicht. Vielmehr können die Daten auf den externen Geräten auch weiterverarbeitet werden, oder es kann dort auf einfache Weise eine Benutzerschnittstelle beispielsweise in Form einer Softwareapplikation bereit gestellt werden, an der dem Benutzer eine Auswahl an Gartenpflanzen und/oder Gemüsearten bereit gestellt werden kann. Dieses Auswahlprogramm kann dann weiterhin beispielsweise in Form einer Datenbank oder Lookup-Tabelle günstige Sollwerte für die Bodenfeuchtigkeit oder den Nährstoffgehalt im Boden aufweisen, sowie eine ebenfalls vorzugsweise als Software ausgebildete Vergleichereinrichtung, welche die hinterlegten Sollwerte für die ausgewählte Gartenpflanze oder Gemüseart mit den erfassten Istwerten für die Bodenfeuchtigkeit und/oder den Nährstoffgehalt im Boden vergleicht und das Ergebnis dann zur Ausgabe auf der Ausgabeeinrichtung bzw. auf dem externen Gerät übermittelt.

Das Gartengerät kann die Benutzerschnittstelle beispielsweise in Form eines Touchscreens oder in Form von Auswahltasten oder dergleichen auch lokal am Gerät selbst aufweisen, ebenso das vorstehend genannte Auswahlprogramm und die Vergleichseinrichtung, etwa in Form von auf dem Microcontroller der Recheneinheit ablaufenden Programmroutinen. Hierzu könnte das Gartengerät einen Speicher wie z.B. eine Micro-SD-Karte enthalten. Es wäre aber wie gesagt ebenso denkbar, die Benutzerschnittstelle und die zugehörige Software alternativ oder ergänzend ganz oder in Teilen als Teil des Gartengeräts auf ein externes Gerät auszulagern.

Weiterhin kann das Gartengerät ergänzend zur Erfassung von weiteren das Aussaat- bzw. Pflanzergebnis beeinflussenden Parametern eingerichtet sein. Insbesondere könnte das Gartengerät eine Temperaturerfassungseinrichtung zur Erfassung einer Anzahl der Umgebungstemperatur entsprechender Größen aufweisen, also beispielsweise einen Temperatursensor in Form eines digitalen Thermometers, welcher z.B. über eine Busverbindung an den Microcontroller angeschlossen ist. Denkbar wären auch weitere Sensoren, die beispielsweise die Farbtemperatur des Umgebungslichtes, den pH-Wert des Bodens oder den Luftstrom messen.

Weiterhin kann das Gartengerät eine Lichterfassungseinrichtung zur Erfassung einer Anzahl den Lichtverhältnissen in der Umgebung entsprechender Größen aufweisen, beispielsweise in Form eines Fototransistors, welcher eine Beleuchtungsstärke des auftreffenden Lichts misst und welcher ebenfalls an dem Microcontroller angeschlossen sein kann. Der Microcontroller kann dann aus dem Lichtstrom die Beleuchtungsstärke messen. Ebenfalls denkbar wäre es, die Lichterfassungseinrichtung als Solarzelle auszubilden.

Weiterhin kann das Gartengerät eine Uhreinrichtung zur Bestimmung der Jahreszeit aufweisen. Denn das Saat- bzw. Pflanzergebnis hängt maßgeblich davon ab, dass zur richtigen Jahreszeit gesät bzw. gepflanzt wird.

Wenn das Gartengerät durch diese vorstehend angesprochenen zusätzlichen Einrichtungen ergänzt ist, kann natürlich auch das erfindungsgemäße Saat- bzw. Pflanzverfahren weiter verfeinert werden, wobei nicht nur günstige Sollwerte für die Bodenfeuchtigkeit und den Nährstoffgehalt im Boden bestimmt werden, sondern auch für die Umgebungstemperatur, die Lichtverhältnisse in der Umgebung und/oder die Aussaat bzw. Pflanzjahreszeit, welche dann mit den ermittelten Istwerten verglichen werden können, um noch genauer festlegen zu können, ob die Aussaat bzw. das Pflanzen durchgeführt werden soll.

Im Folgenden wird anhand der beiliegenden Zeichnungen eine vorteilhafte Ausführungsform der Erfindung näher erläutert.

Es zeigen:
- Figur 1: eine perspektivische Ansicht einer Gartenschaufel gemäß einer vorteilhaften Ausführungsform der Erfindung;
- Figur 2: eine Explosionsdarstellung der in der Fig. 1 gezeigten Gartenschaufel ;
- Figur 3: eine weitere Explosionsdarstellung der in der Fig. 1 gezeigten Gartenschaufel; und die
- Figuren 4-6: Schaltpläne der in den Figuren 1-3 gezeigten Gartenschaufel.

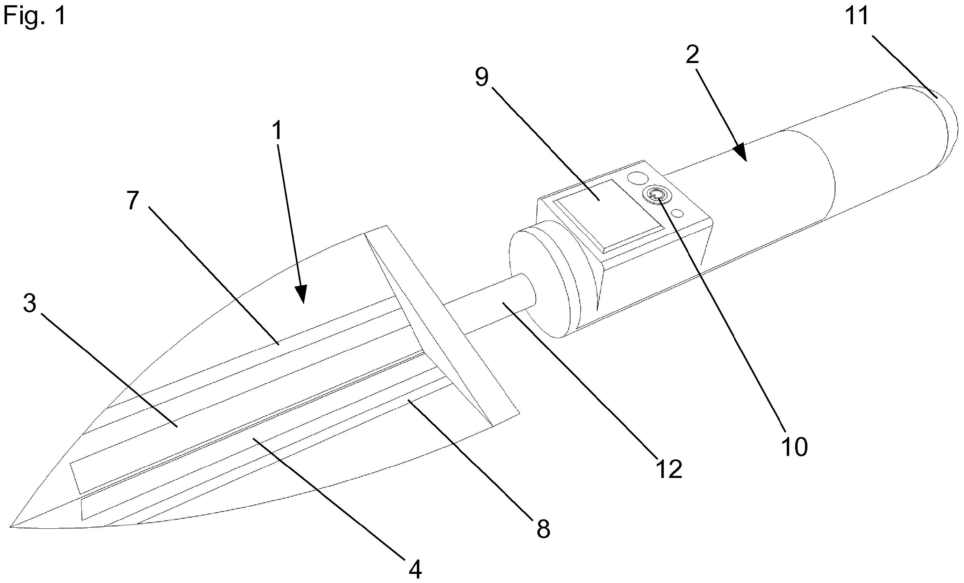

Die Figur 1 zeigt eine Gartenschaufel mit der es möglich ist, während dem Arbeiten die Feuchte und den Nährstoffgehalt des Bodens oder von Pflanzen- bzw. Blumenerde zu messen sowie die Umgebungstemperatur und die Lichtverhältnisse zu bestimmen, so dass man unmittelbar Indikatoren erhält, ob das Pflanz- oder Saatgut an dieser Stelle mit ausreichend Wasser und Nährstoffen versorgt ist und ob die Licht- und Temperaturverhältnisse dem Pflanz- oder Saatgut entsprechen.

Hierzu wird die elektronische Gartenschaufel an einer ausgewählten Stelle in den Boden bzw. in die Erde gesteckt. Die elektronische Gartenschaufel misst nun Nährstoffgehalt und Feuchte des Bodens bzw. der Erde, Temperatur, Lichtverhältnisse der Umgebung und etwaige andere Parameter und teilt dann anhand der Daten und des aktuellen Datums einer nicht gezeigten internen Echtzeituhr dem Benutzer über das Display oder einem anderen Benutzerinterface mit, ob die Jahreszeit und die ausgewählte Stelle für die gewählte Pflanze oder Gemüseart geeignet ist oder nicht. Ist die ausgewählte Stelle und Jahreszeit geeignet, kann das Pflanz- oder Saatgut dort eingebracht werden.

Die Gartenschaufel weist ein Schaufelblatt 1 auf, welches vorzugsweise aus glasfaserverstärkten Kunststoff gefertigt ist, da dieser sich gut als Dielektrikum eignet und hohe Stabilität und Abriebfestigkeit aufweist. Auf der Oberseite des Schaufelblattes 1 sind vier Elektroden 3, 4, 7, 8 aufgebracht. Es wäre aber ebenso denkbar, die Elektroden auf der Unterseite des Schaufelblatts anzubringen. Die Elektroden 3, 4, 7, 8 können aufgedampft, aufgeklebt, aufgedruckt oder durch sonstige mechanische oder chemische Verfahren aufgebracht sein.Von den vier Elektroden 3, 4, 7, 8 dienen zwei als Kondensatorelektroden bezeichnete erste Elektroden 3, 4 dazu, um die Feuchte des Bodens zu messen. Die beiden weiteren, äußeren, als Leitfähigkeitsmesselektroden bezeichneten Elektroden 7, 8 dienen zur Messung des Nährstoffgehalts des Bodens.

Je mehr Nährstoffionen sich im Boden befinden, desto größer ist die elektrische Leitfähigkeit. Um elektrochemische Korrosion der Leitfähigkeitsmesselektroden 7, 8 zu vermeiden, sind diese beiden außenliegenden Leitfähigkeitsmesselektroden 7, 8 vorzugsweise aus Chemisch Nickel/Sudgold (Electroless Nickel Immersion Gold, ENIG) gefertigt, ein Verfahren, das heute bereits bei Leiterbahnen auf Platinen angewandt wird mit einer typischen Gold-Schicht von 0,05 - 0,1 µm und Nickel-Schicht von 4 - 7 µm.

Die Sudgold-Schicht verhindert die Oxidation des Nickels. So soll verhindert werden, dass sich die Leitfähigkeitsmesselektroden 7, 8 zersetzen und für die Pflanzen giftige Ionen freisetzen.

Die beiden inneren Kondensatorelektroden 3, 4 bilden einen Plattenkondensator. Diese beiden Kondensatorelektroden 3, 4 sind über deren komplette Oberfläche luft- und feuchtigkeitsdicht versiegelt, um zu verhindern, das Wasser oder Erde oder Luft direkt an die Elektrodenflächen gelangen kann. Sie dienen zur Ermittlung der Bodenfeuchte. Da diese Kondensatorelektroden 3, 4 versiegelt und dadurch keiner Korrosion oder Oxidation ausgesetzt sind, muss ihr Elektrodenmaterial nicht aufwendig oberflächenbehandelt werden oder aus teuren Elementen oder Legierungen bestehen. Es kann beispielsweise Kupfer oder ähnliches verwendet werden. Der feuchte Boden bzw. die feuchte Erde dient hier als Dielektrikum, das die Kapazität des aus den beiden Elektroden 3, 4 gebildeten Plattenkondensators beeinflusst.

Da das Schaufelblatt 1 und vor allem die freiliegenden äußeren Elektroden 7, 8 durch die Gartenarbeit, die damit verrichtet werden kann, abgenutzt werden, ist das Schaufelblatt 1 so gestaltet, dass sich dieses ohne Spezialwerkzeug austauschen lässt. Dadurch eröffnet sich zudem die Möglichkeit, Schaufelblätter unterschiedlicher Form einzusetzen und als Zubehör anzubieten.

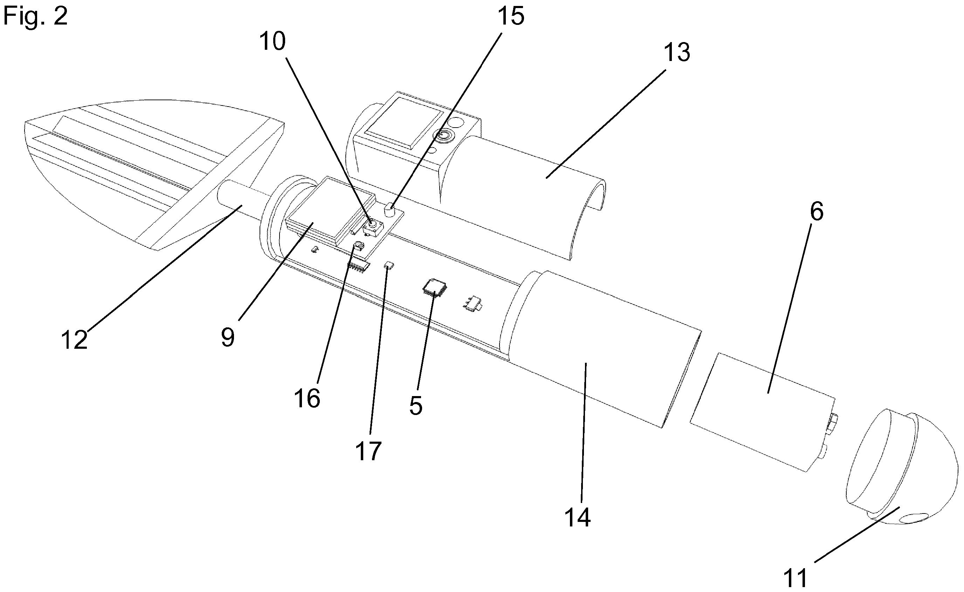

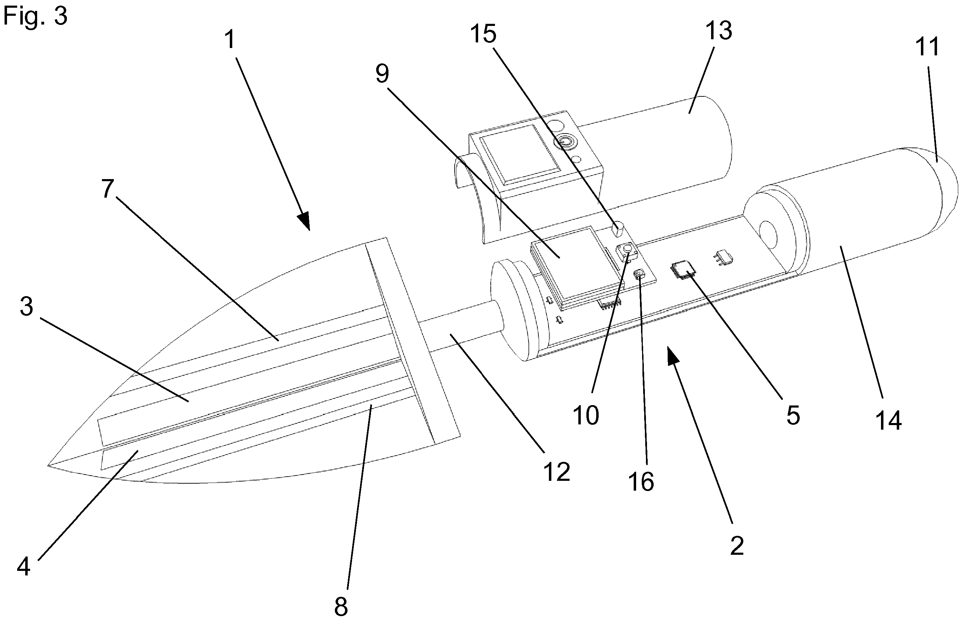

Neben einem EIN/AUS-Schalter 10, einem OLED- oder LCD-Display 9 und einem Microcontroller 5 befinden sich bei der gezeigten Gartenschaufel an oder in einem Handgriffabschnitt 2 drei weitere Sensoren (siehe Fig. 2 und 3) - ein 3-Achsen-Beschleunigungssensor 17, ein Fototransistor 16 und ein Temperatursensor 15. Der 3-Achsen-Beschleunigungssensor 17 misst die Neigung der elektronischen Gartenschaufel. Die analogen oder digitalen und vorab gefilterten Werte der 3 Achsen werden einem Microcontroller 5 zugeführt, der die Position der elektronischen Gartenschaufel ermittelt.

Je nach Neigung werden die auf dem OLED- oder LCD-Display 9 angezeigten Werte gedreht, so dass der Benutzer die Werte, egal in welcher Position sich die elektronische Gartenschaufel gerade befindet, ablesen kann. Ein ähnliches Prinzip findet man heute bereits bei den meisten sogenannten Smartphones. Zudem wäre es möglich, den 3-Achsen-Beschleunigungssensor 17 als Benutzerschnittstelle zu nutzen.

Der Fototransistor 16 misst die Beleuchtungsstärke, also welcher Anteil vom Lichtstrom auf einem Quadratmeter Fläche des beleuchteten Objekts ankommt.

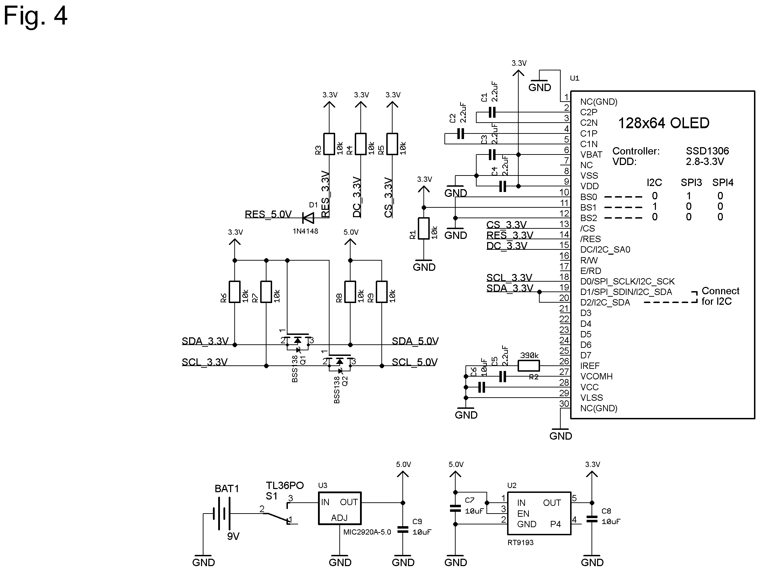

Die Figuren 4-6 zeigen ein mögliches Beispiel eins Schaltplanes für die vorstehend beschriebene Gartenschaufel ohne die erwähnte interne Echtzeituhr und Benutzerschnittstelle, die wie erwähnt beispielsweise aus Tasten,Trackball, Miniatur-Joystick oder Touchscreen bestehen kann.

Fig. 4 zeigt als Beispiel für das Display 9 ein 128 x 64 OLED-Display, hier im Schaltplan bezeichnet als U1, das so konfiguriert ist, dass die Kommunikation über einen I2C-Bus läuft, zudem für den üblichen 5-V-Microcontroller 5, hier im Schaltplan bezeichnet als U5, die notwendigen Pegelwandler des 3,3 V I2C-Busses und der 3,3 V Reset-Leitung sowie die notwendigen Spannungsversorgungen für die komplette Schaltung.

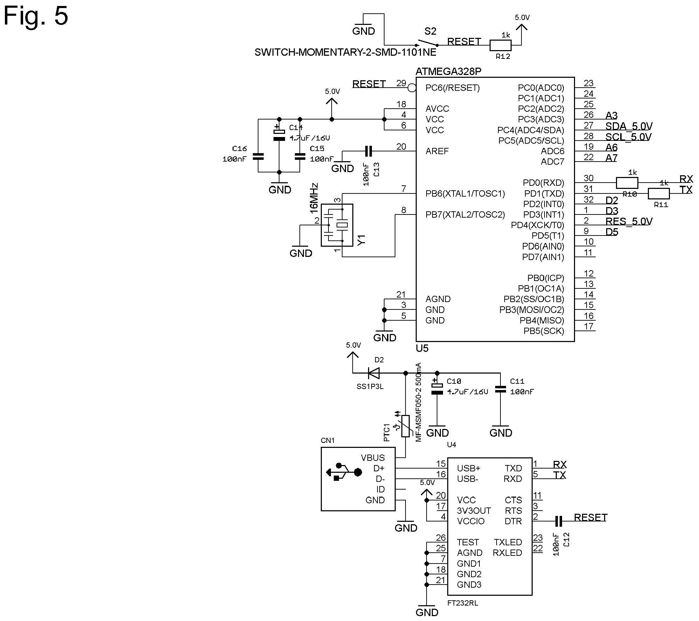

Fig. 5 zeigt als weiteres Beispiel einen typischen 5-V-Microcontroller U5 und dessen Peripherie. In der Abbildung verfügt der Microcontroller U5 über eine USB-Schnittstelle gebildet aus CN1 und dem USB/Seriell-Umsetzer U4, um diesen zu programmieren und um zu debuggen. Dies muss aber nicht notwendigerweise der Fall sein. Programmierung und Debugging des Microcontrollers U5 könnte auch über eine SPI-Schnittstelle oder ähnliches erfolgen, so dass CN1 und U4 dann entfallen könnten.

Der die Kondensatorelektroden 3, 4 (im Schaltplan: PROBE 1, PROBE 2) aufweisende Plattenkondensator bildet zusammen mit einem Schmitt-Trigger IC2 vom Typ 74HC14 einen Oszillator und somit insgesamt die Feuchtigkeitserfassungseinrichtung. Je nach Fläche des Kondensators liegt die Frequenz zwischen einigen 100 kHz und mehreren MHz. Der Oszillator selbst ist ein RC-Oszillator, wobei jedoch die eine Kondensatorelektrode nicht wie üblich auf GND sondern auf Signalpegel liegt, um Störungen welche über die Masseleitung einstreuen könnten, zu minimieren. Je feuchter nun der Boden oder die Erde, desto größer die Kapazität des Kondensators und desto niedriger die Frequenz des Oszillators. Das Ausgangssignal des Oszillators wird einem Digital-Eingang des Microcontrollers U5 zugeführt, der die Frequenz des Oszillators misst und daraus die Bodenfeuchte errechnet.

Die Nährstofferfassungseinrichtung umfasst neben den beiden Leitfähigkeitsmesselektroden 7, 8, die im Schaltplan als PROBE3 und PROBE4 zu entnehmen sind, ebenfalls weitere elektronische Bauteile. Eine der beiden Leitfähigkeitsmesselektroden 7, 8, bzw. im Schaltplan PROBE3 oder PROBE4 ist mit der Basis eines npn-Transistors Q5 verbunden, die andere über deinen Vorwiderstand R19 mit der positiven Versorgungsspannung. Je leitfähiger der Boden, desto mehr leitet der Transistor Q5. Der Transistor Q5 , der hier selbst wie ein Widerstand fungiert, bildet mit dem Widerstand an einem Emitter R17 einen Spannungsteiler. Das Signal wird einem Analog-Eingang des Microcontrollers 5, bzw. im Schaltplan U5 zugeführt. Dieser misst die Spannung und errechnet daraus den Nährstoffgehalt des Bodens. Um die Lebensdauer der Elektroden PROBE 3, PROBE 4, zusätzlich zu erhöhen, sind sie nicht ständig mit der Versorgungsspannung verbunden. Über einen p-Kanal MOSFET Q4 wird der aus den Elektroden PROBE3 und PROBE4 gebildete Sensor nur dann vom Microcontroller U5 aktiviert, wenn im Programmablauf ein Befehl zu Messung des Nährstoffgehalts gegeben wird.

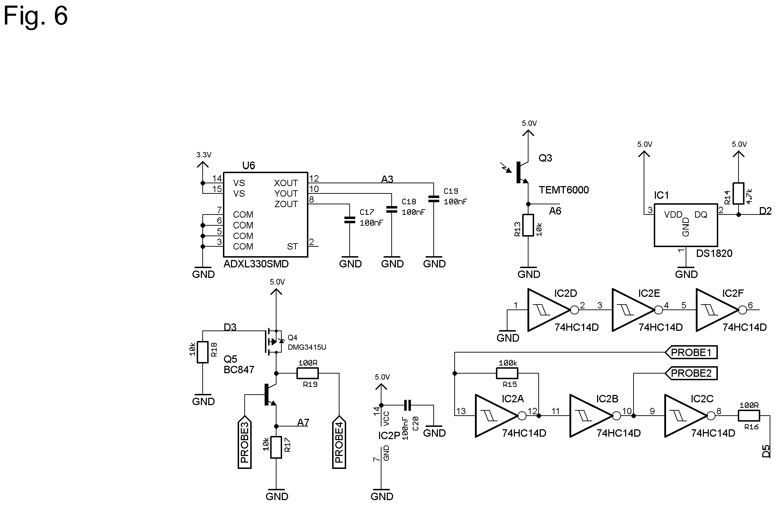

Fig. 6 zeigt noch einmal die fünf Sensoren der Gartenschaufel: Den aus drei Tiefpassfilter-Kondensatoren C17 bis C19 bestehenden 3-Achsen-Beschleunigungssensor 17, hier im Schaltplan U6, den aus dem Fototransistor 16, hier im Schaltplan Q3 und dem Widerstand R13 bestehenden Umgebungslichtsensor, den Temperatursensor 15 bestehend aus IC1 und R11, den aus den weiteren Elektroden PROBE3 und PROBE4, dem npn-Transistor Q5, dem p-Kanal MOSFET Q5 und den Widerständen R17 bis R19 bestehenden Nährstoffsensor, sowie den aus den ersten Elektroden PROBE1 und PROBE2, dem Schmitt-Trigger IC2 und den Widerständen R15 bis R16 bestehenden kapazitiven Feuchtigkeitssensor.

Der Fototransistor Q3 und der Widerstand R13 sind als Spannungsteiler beschaltet. Das Signal wird einem analogen Eingang des Microcontroller U5 zugeführt. Je größer der Anteil des Lichtstroms, desto größer die Spannung am Ausgang des Spannungsteilers. Aus der gemessenen Spannung errechnet der Microcontroller U5 dann die Beleuchtungsstärke. Der Temperatursensor IC1 ist beispielsweise wie hier dargestellt ein digitales Thermometer mit einer programmierbaren Auflösung von 9-12 Bit, einem Messbereich von -55°C bis +125°C und einer Toleranz von ±0.5°C im Bereich von -10°C bis +85°C. Der Temperatursensor IC1 misst die Umgebungstemperatur und kommuniziert mit dem Microcontroller U5 über den sogenannten Eindraht-Bus.

Gespeist wird die elektronische Gartenschaufel durch eine handelsübliche 9-V-Blockbatterie 6, hier im Schaltplan BAT1 oder ähnliche kompakte Batterien oder Akkumulatoren. Die Batterie BAT1 kann vom hinteren Ende des Handgriffs 2 entnommen bzw. getauscht werden, wenn sie entladen ist. Dazu muss vorher die Verschlusskappe 11, die mit einem Gewinde oder sonstigem Verschlussverfahren versehen ist, entfernt werden. Die Verschlusskappe 11 sowie der Handgriff 2 selbst sind wasserdicht ausgeführt, so dass kein Wasser oder Schmutz in das Innere gelangen und die Elektronik beschädigen kann.

Auch denkbar wäre, die Batterie zum Laden nicht entnehmen zu müssen. Die elektronische Gartenschaufel müsste dann über eine entsprechende Anschlussbuchse für ein Ladegerät verfügen oder über ein kontaktloses Akku-Ladesystem, wie es heute beispielsweise bereits für elektrische Zahnbürsten üblich ist.

Die Elektronik ist dabei in einer vom Batteriefach separaten Kammer im Handgriffabschnitt 2 untergebracht, welcher dafür einen Verschlussdeckel 13 aufweist. Aus dieser Kammer sind Leitungen zum Schaufelblatt 1 geführt, und zwar durch einen hohlen Verbindungsschaft 12 des Handgriffabschnitts 2. Abwandlungen und Modifikationen der gezeigten Ausführungsform sind möglich, ohne den Rahmen der Erfindung, wie sie in den beiliegenden Ansprüchen definiert wird, zu verlassen.

## Patentansprüche

1. Gartengerät zur Bodenbearbeitung, mit einem Handgriffabschnitt (2), einem daran angebrachten Bodenkontaktabschnitt, beispielsweise einem Schaufelblatt (1), einer Stromversorgung (6), einer Anzahl Erfassungseinrichtungen (3, 4, 7, 8) zur Erfassung einer Anzahl von Bodeneigenschaften entsprechender Größen, einer Recheneinheit (5) zur Berechnung der Bodeneigenschaften aus der den Bodeneigenschaften entsprechenden Anzahl von Größen, und einer Ausgabeeinrichtung (9) zur Ausgabe der Bodeneigenschaften und/oder auf den Bodeneigenschaften basierenden Angaben, wobei der Bodenkontaktabschnitt Elektroden (3, 4, 7, 8) trägt, über welche die Anzahl der den Bodeneigenschaften entsprechenden Größen erfasst werden, und wobei eine der Anzahl Erfassungseinrichtungen eine Nährstofferfassungseinrichtung zur Erfassung einer Anzahl einem Nährstoffgehalt im Boden entsprechender Größen ist, und die Recheneinheit (5) zur Berechnung des Nährstoffgehalts im Boden aus der dem Nährstoffgehalt im Boden entsprechenden Anzahl Größen eingerichtet ist und die Ausgabeeinrichtung (9) zur Ausgabe des Nährstoffgehalts im Boden,
**dadurch gekennzeichnet, dass**
der Bodenkontaktabschnitt (1) aus faserverstärktem Kunststoff wie beispielsweise glasfaserverstärktem Kunststoff gefertigt ist, und
die Nährstofferfassungseinrichtung eine elektrische Leitfähigkeit des Bodens als Maß für den Nährstoffgehalt erfasst und zur Leitfähigkeitserfassung zumindest zwei der Elektroden (7, 8) umfasst, die voneinander beabstandet auf dem Bodenkontaktabschnitt angebracht sind, wobei
diese Leitfähigkeitsmesselektroden (7, 8) eine als Chemisch Nickel auf den Bodenkontaktabschnitt abgeschiedenen Schicht umfassen oder daraus bestehen.

2. Gartengerät nach Anspruch 1, **dadurch gekennzeichnet, dass** die Elektroden (3, 4, 7, 8) auf der Oberfläche des Bodenkontaktabschnitts (1) aufgebracht sind.

3. Gartengerät nach Anspruch 1 oder 2, **dadurch gekennzeichnet, dass** das Gartengerät eine Feuchtigkeitserfassungseinrichtung zur Erfassung einer Anzahl einer Bodenfeuchtigkeit entsprechender Größen aufweist, wobei die Recheneinheit (5) zur Berechnung der Bodenfeuchtigkeit aus der der Bodenfeuchtigkeit entsprechenden Anzahl von Größen eingerichtet ist und die Ausgabeeinrichtung zur Ausgabe der Bodenfeuchtigkeit und/oder auf der Bodenfeuchtigkeit basierenden Angaben, und wobei die Feuchtigkeitserfassungseinrichtung zumindest zwei der Elektroden (3, 4) aufweist, die nach Art eines Plattenkondensators voneinander beabstandet auf dem Bodenkontaktabschnitt angebracht sind, wobei eine Kapazität des Plattenkondensators mit Erde als Dielektrikum beeinflusst wird, wenn die Erde mit dem Bodenkontaktabschnitt im Bereich zwischen diesen Kondensatorelektroden (3, 4) kontaktiert wird.

4. Gartengerät nach Anspruch 1, 2 oder 3, **dadurch gekennzeichnet, dass** die Recheneinheit dazu eingerichtet ist, aus der Kapazität des Plattenkondensators und/oder aus einer aus der Kapazität des Plattenkondensators abgeleiteten Größe als Eingangsgröße eine die Bodenfeuchtigkeit wiedergebende Ausgangsgröße zu errechnen.

5. Gartengerät nach Anspruch 1, 2 oder 3, **dadurch gekennzeichnet, dass** die Feuchtigkeitserfassungseinrichtung einen mit dem Plattenkondensator zu einer Oszillatorschaltung, insbesondere zu einem Kippschwinger zusammengeschalteten Schwingungserzeuger aufweist, insbesondere einen Schmitt-Trigger, wobei die Frequenz der erzeugten Schwingung und/oder die Kapazität des Plattenkondensators der Recheneinrichtung (5) als Eingangsgröße für die Bodenfeuchtigkeit zugeführt wird.

6. Gartengerät nach einem der vorhergehenden Ansprüche, **dadurch gekennzeichnet, dass** die beiden Kondensatorelektroden (3, 4) der Feuchtigkeitserfassungseinrichtung aus einem leitfähigen Material wie Kupfer oder einer Kupferlegierung bestehen und gegenüber der Umgebung luft- und feuchtigkeitsdicht versiegelt auf den Bodenkontaktabschnitt aufgebracht sind.

7. Gartengerät nach einem der vorhergehenden Ansprüche, **dadurch gekennzeichnet, dass** der Bodenkontaktabschnitt (1) auswechselbar, insbesondere werkzeuglos auswechselbar, beispielsweise über einen Bajonettverschluss an dem Handgriffabschnitt (2) angebracht ist.

8. Gartengerät nach einem der vorhergehenden Ansprüche, **dadurch gekennzeichnet, dass** der Handgriffabschnitt einen Stiel (2) umfasst, in dem ein Mikrocontroller (5) der Recheneinheit und eine Anzahl vorzugsweise aufladbarer Batterien (6) der Stromversorgung untergebracht sind, wobei an dem Stiel ein EIN-/AUS-Schalter (10) und vorzugsweise auch ein Display (9) der Ausgabeeinrichtung angeordnet ist.

9. Gartengerät nach Anspruch 7 oder 8, **dadurch gekennzeichnet, dass** eine Anzahl unterschiedlich geformter, jeweils mit den beiden Kondensatorelektroden (3, 4) versehener und auf den Handgriffabschnitt passender Bodenkontaktabschnitte, beispielsweise ein Schaufelblatt (1), eine Kelle und/oder eine Hacke umfasst sind.

10. Gartengerät nach einem der vorhergehenden Ansprüche, **dadurch gekennzeichnet, dass** das Gartengerät eine Temperaturerfassungseinrichtung (15) zur Erfassung einer Anzahl der Umgebungstemperatur entsprechender Größen, eine Lichterfassungseinrichtung (16) zur Erfassung einer Anzahl den Lichtverhältnissen in der Umgebung entsprechender Größen und/oder eine Uhreinrichtung zur Bestimmung der Jahreszeit aufweist, wobei die Recheneinheit zur Berechnung der Umgebungstemperatur, der Lichtverhältnisse in der Umgebung und/oder der Jahreszeit eingerichtet ist, und wobei die Ausgabeeinrichtung zur Ausgabe der Umgebungstemperatur, der Lichtverhältnisse in der Umgebung und/oder der Jahreszeit eingerichtet ist.

11. Gartengerät nach einem der Ansprüche 2 bis 10, **dadurch gekennzeichnet, dass** die beiden Kondensatorelektroden (3, 4) zwischen den beiden Leitfähigkeitsmesselektroden (7, 8) angeordnet sind.

12. Gartengerät nach einem der vorhergehenden Ansprüche **dadurch gekennzeichnet, dass** die aus Chemisch Nickel bestehende Schicht der beiden Leitfähigkeitsmesselektroden (7, 8) mit einer Goldschicht bedeckt ist.

13. Gartengerät nach einem der vorhergehenden Ansprüche, **dadurch gekennzeichnet, dass** die Ausgabevorrichtung ein Kommunikationsmodul, insbesondere ein Bluetooth-Funkmodul zur drahtlosen Datenübertragung auf ein externes Gerät wie z.B. ein Smartphone aufweist.

14. Gartengerät nach einem der vorhergehenden Ansprüche, **dadurch gekennzeichnet, dass** das Gartengerät eine Benutzerschnittstelle aufweist, beispielsweise einen Touchscreen, ein Auswahlprogramm an per Benutzerschnittstelle auswählbaren Gartenpflanzen und/oder Gemüsearten, in dem günstige Sollwerte (Bodenfeuchtigkeit, Nährstoffgehalts im Boden, Umgebungstemperatur, Lichtverhältnisse in der Umgebung und/oder Aussaat- bzw Pflanzjahreszeit) für die Gartenpflanzen und/oder Gemüsearten hinterlegt sind, sowie eine Vergleichereinrichtung, insbesondere eine auf dem Mikrocontroller (5) laufende Programmroutine, welche die hinterlegten Sollwerte für die ausgewählte Gartenpflanze oder Gemüseart mit den erfassten Istwerten für Bodenfeuchtigkeit, Nährstoffgehalts im Boden, Umgebungstemperatur, Lichtverhältnisse in der Umgebung und/oder Jahreszeit vergleicht und das Ergebnis zur Ausgabe auf der Ausgabeeinrichtung übermittelt.

15. Saat- bzw. Pflanzverfahren, wobei für eine auszusäende oder zu pflanzende Gartenpflanze und/oder Gemüseart günstige Sollwerte für eine Anzahl von Bodeneigenschaften wie Bodenfeuchtigkeit, und eines Nährstoffgehalts im Boden, sowie vorteilhaft Umgebungstemperatur, Lichtverhältnisse in der Umgebung und/oder Aussaat- bzw Pflanzjahreszeit ermittelt werden, an einer zur Aussaat bzw. zum Pflanzen angedachten Stelle Istwerte für die Anzahl von Bodeneigenschaften sowie vorteilhaft der Umgebungstemperatur, der Lichtverhältnisse in der Umgebung und/oder der Jahreszeit ermittelt werden, dann die Sollwerte mit den Istwerten verglichen werden, und, falls der Vergleich positiv ausfällt, die Aussaat bzw. das Pflanzen durchgeführt wird und sonst nicht, **dadurch gekennzeichnet, dass** zumindest das Ermitteln der Istwerte, vorteilhaft auch das Ermitteln der Sollwerte, der Vergleich der Sollwerte mit den Istwerten sowie die Aussaat bzw. das Pflanzen mit Hilfe eines Gartengeräts nach einem der vorhergehenden Ansprüche durchgeführt wird.

## Claims

1. Gardening device for soil cultivation, comprising a handle section (2), a ground contact section attached thereto, for example a blade (1), a power supply (6), a number of detection devices (3, 4, 7, 8) for detecting a number of variables corresponding to soil properties, a computing unit (5) for calculating the soil properties from the number of variables corresponding to the soil properties, and an output device (9) for outputting the soil properties and/or soil properties based information, wherein the ground contact section (1) carries electrodes (3, 4, 7, 8), via which the number of the variables corresponding to the soil properties are detected, and wherein one of the number of detection devices is a nutrient detection device for detecting a number of variables corresponding to the nutrient content in the soil, and the computing unit (5) is designed for calculating the nutrient content in the soil from the number of variables corresponding to the nutrient content in the soil and the output device (9) for outputting the nutrient content in the soil, **characterized in that** the ground contact section (1) is made of fiber-reinforced plastic such as glass-fiber-reinforced plastic, and the nutrient detection device detects an electrical conductivity of the soil as a measure of the nutrient content and comprises at least two of the electrodes (7, 8) for conductivity detection, which are mounted spaced apart on the ground contact section, wherein these conductivity measuring electrodes (7, 8) comprise or consist of a layer deposited as electroless nickel on the ground contact section.

2. Gardening device according to claim 1, **characterized in that** the electrodes (3, 4, 7, 8) are applied to the surface of the ground contact section (1).

3. Gardening device according to claim 1 or 2, **characterized in that** the gardening device has a moisture detection device for detecting a number of variables corresponding to soil moisture, wherein the computing unit (5) is designed for calculating the soil moisture from the number of variables corresponding to the soil moisture and the output device 5) for outputting the soil moisture and/or data based on the soil moisture, and wherein the moisture detection device (9) has at least two of the electrodes (3, 4), which are mounted in the manner of a plate capacitor spaced from each other on the ground contact section, such that a capacitance of the plate capacitor is influenced with the soil as a dielectric when the soil is contacted with the ground contact section in the region between these capacitor electrodes (3, 4).

4. Gardening device according to claim 1, 2 or 3, **characterized in that** the computing unit is designed to calculate an output variable representing the soil moisture from the capacitance of the plate capacitor and/or a variable derived from the capacitance of the plate capacitor as an input variable.

5. Gardening device according to claim 1, 2 or 3, **characterized in that** the moisture detection device comprises a vibration generator, in particular a Schmitt trigger, which is interconnected to the plate capacitor to form an oscillator circuit, in particular tilting oscillator, wherein the frequency of the generated vibration and/or the capacitance of the plate capacitor is supplied to the computing device (5) as an input variable for the soil moisture.

6. Gardening device according to one of the preceding claims, **characterized in that** the two capacitor electrodes (3, 4) of the moisture detection device consist of a conductive material such as copper or a copper alloy and are applied to the ground contact section in an air-tight and moisture-tight sealed manner against the ambient environment.

7. Gardening device according to one of the preceding claims, **characterized in that** the ground contact section (1) is interchangeably attached to the handle section (2), in particular interchangeably without tools, e.g. via a bayonet lock.

8. Gardening device according to one of the preceding claims, **characterized in that** the handle section comprises a handle (2) in which a microcontroller (5) of the computing unit and a number of preferably rechargeable batteries (6) of the power supply are housed, wherein on the handle an ON/OFF switch (10) and preferably also a display (9) of the output device are arranged.

9. Gardening device according to claim 7 or 8, **characterized in that** it comprises a number of differently shaped ground contact sections, such as a blade (1), a trowel and/or a hoe, in each case provided with the two capacitor electrodes (3, 4) and matching the handle section.

10. Gardening device according to one of the preceding claims, **characterized in that** the gardening device has a temperature detecting device (15) for detecting a number of variables corresponding to the ambient temperatures, a light detecting device (16) for detecting a number of variables corresponding to the light conditions in the environment, and/or a clock device for determining the season, wherein the computing unit is designed for computing the ambient temperature, the lighting conditions in the environment and/or the season, and wherein the output device is designed for outputting the ambient temperature, the lighting conditions in the environment and/or the season.

11. Gardening device according to one of the claims 2 to 10, **characterized in that** the two capacitor electrodes (3, 4) are arranged between the two conductivity measuring electrodes (7, 8).

12. Gardening device according to one of the preceding claims, **characterized in that** the layer consisting of electroless nickel of the two conductivity measuring electrodes (7, 8) is covered with a gold layer.

13. Gardening device according to one of the preceding claims, **characterized in that** the output device is a communication module, in particular a Bluetooth radio module for wireless data transmission to an external device such as a smartphone.

14. Gardening device according to one of the preceding claims, **characterized in that** the gardening device has a user interface, e.g. a touchscreen, a selection program of garden plants and/or vegetable species selectable via a user interface, in which the favorable target values (soil moisture, nutrient content in the soil, ambient temperature, lighting conditions in the environment and/or sowing or planting season) for the garden plants and/or vegetable species are stored, as well as a comparator device, in particular a program routine running on the microcontroller (5), which compares the stored target values for the selected garden plant or vegetable species with the detected actual values for soil moisture, nutrient content in the soil, ambient temperature, ambient light conditions and/or season and transmits the result for output on the output device.

15. Sowing or planting method, wherein favorable target values for a number of soil properties such as soil moisture, and a nutrient content in the soil, and advantageous ambient temperature, lighting conditions in the environment and/or sowing or planting season are determined for a garden plant and/or vegetable species to be sowed or planted, actual values for the number of soil properties and, advantageously, the ambient temperature, the ambient light conditions and/or the season are determined at a location intended for sowing or planting, then the target values are compared with the actual values and, if the comparison is positive, the sowing or planting is carried out and not otherwise, **characterized in that** at least the determination of the actual values, advantageously also the determination of the target values, the comparison of the target values with the actual values and the sowing or planting is performed with the aid of a gardening device according to one of the preceding claims.

## Revendications

1. Outil de jardinage destiné à la culture du sol, comprenant une portion de manche (2), une portion de contact avec le sol y attachée, par exemple une lame de pelle (1), une alimentation électrique (6), un nombre de dispositifs de détection (3, 4, 7, 8) destinés à détecter un nombre de grandeurs correspondant à des propriétés du sol, une unité de calcul (5) destinée à calculer les propriétés du sol à partir du nombre de grandeurs correspondant aux propriétés du sol, ainsi qu'un dispositif de sortie (9) destiné à délivrer les propriétés du sol et/ou des informations basées sur les propriétés du sol, dans lequel ladite portion de contact avec le sol (1) porte des électrodes (3, 4, 7, 8) au moyen desquelles est détecté le nombre de grandeurs correspondant aux propriétés du sol, et dans lequel l'un dudit nombre de dispositifs de détection est un dispositif de détection d'éléments nutritifs destiné à détecter un nombre de grandeurs correspondant à une teneur en éléments nutritifs dans le sol, et ladite unité de calcul (5) est agencée pour calculer la teneur en éléments nutritifs dans le sol à partir du nombre de grandeurs correspondant à la teneur en éléments nutritifs dans le sol, et ledit dispositif de sortie (9) est agencé pour délivrer la teneur en éléments nutritifs dans le sol,
**caractérisé par le fait que**
la portion de contact avec le sol (1) est réalisée en matière plastique renforcée de fibres, telle que, par exemple, une matière plastique renforcée de fibres de verre, et
le dispositif de détection d'éléments nutritifs détecte une conductivité électrique du sol en tant que mesure de la teneur en éléments nutritifs et comprend, pour la détection de conductivité, au moins deux des électrodes (7, 8) qui sont montées à distance l'une de l'autre sur ladite portion de contact avec le sol, dans lequel
ces électrodes de mesure de conductivité (7, 8) comprennent ou se composent d'une couche qui est déposée en tant que nickel chimique sur la portion de contact avec le sol.

2. Outil de jardinage selon la revendication 1, **caractérisé par le fait que** les électrodes (3, 4, 7, 8) sont montées sur la surface de la portion de contact avec le sol (1).

3. Outil de jardinage selon la revendication 1 ou 2, **caractérisé par le fait que** l'outil de jardinage comprend un dispositif de détection d'humidité destiné à détecter un nombre de grandeurs correspondant à une humidité du sol, dans lequel ladite unité de calcul (5) est agencée pour calculer l'humidité du sol à partir du nombre de grandeurs correspondant à l'humidité du sol et ledit dispositif de sortie (9) est agencé pour délivrer l'humidité du sol et/ou des informations basées sur l'humidité du sol, et dans lequel le dispositif de détection d'humidité comprend au moins deux des électrodes (3, 4) qui sont montées sur ladite portion de contact avec le sol en étant espacées les unes des autres à la manière d'un condensateur à plaques, une capacité du condensateur à plaques étant influencée par la terre en tant que diélectrique lorsque la terre est mise en contact avec la portion de contact avec le sol dans la zone située entre ces électrodes de condensateur (3, 4).

4. Outil de jardinage selon la revendication 1, 2 ou 3, **caractérisé par le fait que** l'unité de calcul est agencée pour calculer, à partir de la capacité du condensateur à plaques et/ou à partir d'une grandeur dérivée de la capacité du condensateur à plaques, en tant que grandeur d'entrée, une grandeur de sortie reproduisant l'humidité du sol.

5. Outil de jardinage selon la revendication 1, 2 ou 3, **caractérisé par le fait que** le dispositif de détection d'humidité présente un générateur d'oscillations, en particulier un déclencheur de Schmitt, qui est monté avec le condensateur à plaques de manière à former un circuit d'oscillateur, en particulier un oscillateur de relaxation, dans lequel la fréquence de l'oscillation générée et/ou la capacité du condensateur à plaques est fournie au dispositif de calcul (5) en tant que grandeur d'entrée pour l'humidité du sol.

6. Outil de jardinage selon l'une quelconque des revendications précédentes, **caractérisé par le fait que** les deux électrodes de condensateur (3, 4) du dispositif de détection d'humidité sont réalisées à partir d'un matériau conducteur tel que le cuivre ou d'un alliage de cuivre et sont appliquées sur la portion de contact avec le sol en étant scellées de manière étanche à l'air et à l'humidité par rapport à l'environnement.

7. Outil de jardinage selon l'une quelconque des revendications précédentes, **caractérisé par le fait que** la portion de contact avec le sol (1) est montée de manière remplaçable sur la portion de manche (2), en particulier de manière remplaçable sans outil, par exemple par l'intermédiaire d'une fermeture à baïonnette.

8. Outil de jardinage selon l'une quelconque des revendications précédentes, **caractérisé par le fait que** la portion de manche comprend une tige (2), dans laquelle sont logés un microcontrôleur (5) de l'unité de calcul et un nombre de piles de préférence rechargeables (6) de l'alimentation électrique, dans lequel un commutateur ON/OFF (10) et de préférence également un écran (9) du dispositif de sortie sont agencés, eux aussi, sur la tige.

9. Outil de jardinage selon la revendication 7 ou 8, **caractérisé par le fait qu'**un nombre de portions de contact avec le sol formées de manière différente, munies chacune des deux électrodes de condensateur (3, 4) et s'ajustant sur ladite portion de manche, par exemple une lame de pelle (1), une truelle et/ou une houe, sont comprises.

10. Outil de jardinage selon l'une quelconque des revendications précédentes, **caractérisé par le fait que** l'outil de jardinage comprend un dispositif de détection de température (15) destiné à détecter un nombre de grandeurs correspondant à la température ambiante, un dispositif de détection de lumière (16) destiné à détecter un nombre de grandeurs correspondant aux conditions d'éclairage dans l'environnement, et/ou un dispositif d'horloge destiné à déterminer la saison, dans lequel ladite unité de calcul est agencée pour calculer la température ambiante, les conditions d'éclairage dans l'environnement et/ou la saison, et dans lequel le dispositif de sortie est agencé pour délivrer la température ambiante, les conditions d'éclairage dans l'environnement et/ou la saison.

11. Outil de jardinage selon l'une quelconque des revendications 2 à 10, **caractérisé par le fait que** les deux électrodes de condensateur (3, 4) sont disposées entre les deux électrodes de mesure de conductivité (7, 8).

12. Outil de jardinage selon l'une quelconque des revendications précédentes, **caractérisé par le fait que** la couche se composant de nickel chimique des deux électrodes de mesure de conductivité (7, 8) est recouverte d'une couche d'or.

13. Outil de jardinage selon l'une quelconque des revendications précédentes, **caractérisé par le fait que** le dispositif de sortie est un module de communication, en particulier un module radio Bluetooth pour la transmission de données sans fil à un dispositif externe tel que, par exemple, un smartphone.

14. Outil de jardinage selon l'une quelconque des revendications précédentes, **caractérisé par le fait que** l'outil de jardinage comprend une interface utilisateur, tel qu'un écran tactile, un programme de sélection de plantes de jardin et/ou d'espèces de légume qui peuvent être sélectionné(e)s par l'interface utilisateur, dans lequel sont stockées des valeurs de consigne favorables (l'humidité du sol, la teneur en éléments nutritifs dans le sol, la température ambiante, les conditions d'éclairage dans l'environnement et/ou la saison de semis ou bien de plantation) pour les plantes de jardin et/ou les espèces de légume, ainsi qu'un dispositif comparateur, en particulier une routine de programme exécutée sur le microcontrôleur (5), qui compare les valeurs de consigne stockées pour la plante de jardin ou l'espèce de légume sélectionné(e) aux valeurs réelles détectées pour l'humidité du sol, la teneur en éléments nutritifs dans le sol, la température ambiante, les conditions d'éclairage dans l'environnement et/ou la saison, et transmet le résultat pour le délivrer sur ledit dispositif de sortie.

15. Procédé de semis ou bien de plantation, dans lequel, pour une plante de jardin et/ou espèce de légume à semer ou à planter, des valeurs de consigne favorables pour un nombre de propriétés du sol, telles que l'humidité du sol, et d'une teneur en éléments nutritifs dans le sol, ainsi que, avantageusement, la température ambiante, les conditions d'éclairage dans l'environnement et/ou la saison de semis ou bien de plantation sont déterminées, des valeurs réelles pour le nombre de propriétés du sol ainsi que, avantageusement, de la température ambiante, des conditions d'éclairage dans l'environnement et/ou de la saison sont déterminées sur un point prévu pour le semis ou bien pour la plantation, puis les valeurs de consigne sont comparées aux valeurs réelles et, si la comparaison est positive, le semis ou bien la plantation est effectué(e) et, sinon, n'est pas effectué(e), **caractérisé par le fait qu'**au moins la détermination des valeurs réelles, de préférence également la détermination des valeurs de consigne, la comparaison des valeurs de consigne aux valeurs réelles ainsi que le semis ou bien la plantation sont mis en œuvre au moyen d'un outil de jardinage selon l'une quelconque des revendications précédentes.
